# FASCICULE DE BREVET EUROPEEN

(11) **EP 0 806 471 B1**
(45) Date de publication et mention de la délivrance du brevet: **29.12.1999**
(21) Numéro de dépôt: 97400975.5
(22) Date de dépôt: 30.04.1997
(51) Int. Cl.: C11C 3/04, C07C 67/03, C07C 69/24, C07C 69/52

(54) **Procédé de fabrication d'esters éthyliques d'acides gras**
Verfahren zur Herstellung von Fettsäureäthylestern
Process for the production of fatty acid ethyl esters

(30) Priorité: 07.05.1996 FR 9605820
(43) Date de publication de la demande: 12.11.1997
(73) Titulaire: INSTITUT FRANCAIS DU PETROLE, 92852 Rueil-Malmaison Cedex (FR)
(72) Inventeur: Stern, Robert, 75017 Paris (FR); Hillion, Gérard, 95220 Herblay (FR); Durand, Jean-Pierre, 78400 Chatou (FR); Eisa, Mohammed Neguib, 1220 Vienne (AT)

(56) Documents cités:
- WO-A-94/17027
- FR-A- 2 577 569
- FR-A- 2 696 185
- OLEAGINEUX, vol. 40, no. 3, 1985, FR, pages 147-151, XP002025504 R.C.A. LAGO ET AL.: "Extraction and transesterification of vegetable oils with ethanol"

## Description

L'invention concerne un nouveau procédé de fabrication d'esters éthyliques dérivés de corps gras naturels, ces esters étant utilisables notamment comme carburants pour moteurs Diesel ou comme combustibles domestiques.

On connaît de nombreux procédés pour fabriquer des esters utilisant l'alcool éthylique sec. En général, ces procédés sont analogues à ceux qui utilisent l'alcool méthylique, si ce n'est que l'alcool éthylique doit parfois être évaporé avant la décantation de la glycérine. Par ailleurs, si le séchage de l'alcool éthylique fait l'objet d'une littérature abondante, il reste que les procédés préconisés sont souvent coûteux, surtout à petite échelle. A grande échelle (soit plusieurs milliers de tonnes/an), les coûts de fonctionnement peuvent être réduits, mais les investissements sont très importants.

Dans le domaine de la transestérification des corps gras au moyen d'alcools hydratés (en particulier alcool éthylique hydraté), on ne dispose que de peu de références. Par exemple, le brevet français FR-B-2.577.569, au nom du même déposant, décrit l'utilisation de catalyseurs acides, mais la réaction est terminée d'une part par un séchage de l'alcool éthylique dans une opération qui vise à estérifier l'acide libre, et d'autre part par une catalyse basique. Dans l'opération de séchage de l'alcool, on utilise un tamis moléculaire qu'il convient de régénérer.

Dans ces conditions, la fabrication d'esters éthyliques de corps gras au moyen d'alcool éthylique hydraté apparaît comme une gageure, mais d'un enjeu important, notamment pour les pays en voie de développement. L'objectif semble difficile à atteindre puisqu'il consiste à obtenir un ester éthylique assez pur avec un bon rendement par rapport au corps gras de départ, tout en laissant la possibilité de recycler sans problème l'alcool engagé en excès.

On a maintenant découvert, de manière inattendue, que, en associant plusieurs étapes connues séparément, il était possible d'atteindre l'objectif fixé, c'est-à-dire de concilier les diverses exigences demandées à un procédé qui doit être applicable à une échelle artisanale. Parmi ces exigences, on peut mentionner un investissement qui doit rester faible, en ce qui concerne notamment les composants métalliques des appareillages mis en oeuvre (structures en acier) et la nécessité de recourir le moins possible au vide ou à des opérations sous pression.

Le principe du procédé de l'invention réside dans le fait que l'alcool éthylique utilisé en excès pour obtenir une bonne conversion des esters glycéridiques de l'huile traitée peut être recyclé malgré une concentration en eau supérieure à celle de l'alcool de départ. Cela est rendu possible par l'addition de glycérine qui se comporte comme un desséchant, en retenant davantage l'eau que l'alcool. Les pertes en esters éthyliques entraînés par la glycérine avec les savons formés sont compensées par un recyclage de la phase "esters + acides gras" retransformés partiellement en glycérides.

Ainsi, l'invention propose un procédé de fabrication d'esters éthyliques d'acides gras à partir d'huile ou de graisse végétale ou animale ou d'autres mélanges de glycérides, ce procédé pouvant être défini d'une manière générale par le fait qu'il comprend cinq étapes telles que définies ci-après, en relation avec le schéma qui sera présenté ci-après.

Dans une étape (a), on transestérifie l'huile, la graisse ou le mélange de glycérides de départ par de l'alcool éthylique hydraté en excès en utilisant un catalyseur alcalin.

Dans une étape (b), on ajoute dans le milieu une phase glycérineuse, qui peut être une fraction de la phase glycérineuse B séchée obtenue à l'étape (d) ci-après, et on évapore l'alcool éthylique en excès, de manière à produire deux phases, une phase ester et une phase glycérineuse A, non miscibles, et l'on recycle l'alcool éthylique en excès vers l'étape (a).

Dans une étape (c), on sépare ladite phase glycérineuse A et ladite phase ester, que l'on purifie, de manière à obtenir les esters éthyliques recherchés.

Dans une étape (d), on neutralise ladite phase glycérineuse A et on sépare une phase "acides gras + esters" et une phase glycérineuse B, que l'on sèche, une fraction de la phase glycérineuse B séchée pouvant être retournée vers l'étape (b).

Dans une étape (e), on effectue la glycérolyse de la phase "acides gras + esters" avec une fraction de la phase glycérineuse B séchée et en présence d'un catalyseur alcalin, qui peut être une fraction de la phase glycérineuse A, pour former, après séparation par exemple par filtration, un mélange de glycérides et d'esters, que l'on envoie dans une étape de transestérification telle que (a).

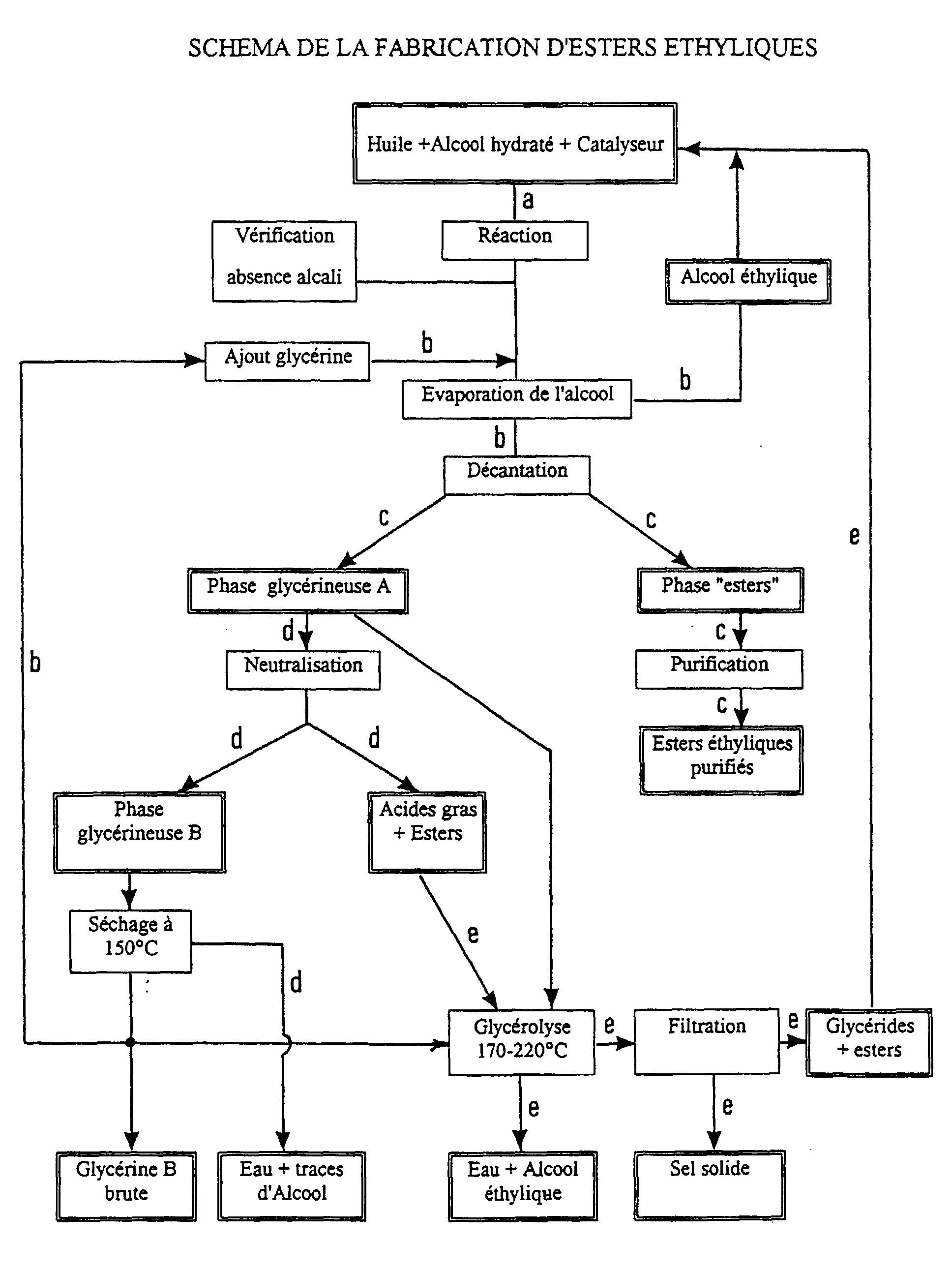

On décrit ci-après plus en détail les différentes étapes du procédé de l'invention.

Dans l'étape (a), on part avantageusement d'une huile neutre pour ne pas augmenter la consommation en catalyseur alcalin qui est déjà plus importante que lorsqu'on opère avec un alcool sec. Les huiles utilisables dans le procédé de l'invention sont toutes les huiles naturelles (végétales ou animales) connues dans les domaines alimentaire et industriel. On peut citer à titre d'exemples les huiles lauriques (huiles de coprah et de palmiste), les huiles riches en acide palmitique (huile de palme, suif), les huiles riches en acide oléique (huiles d'arachide, de colza et d'olive), les huiles riches en acides polyinsaturés (huiles de poisson, de soja, de lin, de noix, de carthame et de tournesol) etc.. Si l'on souhaite utiliser des huiles de friture, trop acides, il y a lieu de réduire au préalable leur acidité par une glycérolyse à une température de 60 à 100°C, en présence d'un catalyseur acide et de glycérine.

La transestérification de l'étape (a) étant effectuée en catalyse basique, la teneur en eau de l'alcool éthylique utilisé joue un grand rôle dans la conversion de l'huile.En effet, plus la teneur en eau est importante, plus il est difficile d'obtenir un ester éthylique avec une bonne pureté à l'issue de l'étape (c). Il y a compétition entre la transestérification, qui requiert un alcoolate alcalin comme catalyseur, et la saponification, qui transforme le catalyseur en savon. La présence d'eau favorise la saponification de l'huile ou des esters éthyliques. Aussi, l'alcool éthylique utilisé présentera en général un taux d'alcool pur de 92 à 99%, le plus souvent de 94 à 97%. On peut en effet dépasser la concentration azéotropique de 96%. Grâce à la présence de glycérine dans l'étape (b) qui comprend une évaporation de l'excès d'alcool éthylique, l'alcool éthylique qui passe en tête est plus sec que l'alcool de départ et on peut sans problème le recycler vers l'étape (a). On peut encore envisager d'ajouter à l'alcool éthylique un autre alcool, l'alcool méthylique étant à cet égard particulièrement intéressant, puisqu'il permet une meilleure conversion en milieu basique et qu'il est en outre un constituant de dénaturant.

On utilise en général une proportion d'alcool éthylique de 30 à 100%, de préférence de 40 à 60%, en poids par rapport à l'huile de départ. Dans ces conditions, pour obtenir une conversion totale de l'huile, il convient d'utiliser une proportion de catalyseur alcalin, exprimé en poids de soude, de 1 à 2% en poids par rapport à l'huile de départ. Le catalyseur peut être choisi par exemple parmi la soude, la potasse et les alcoolates de sodium ou de potassium, tels par exemple que le méthylate ou l'éthylate de sodium. On utilise de préférence la soude en raison de son faible coût. On peut aussi avoir intérêt à utiliser la potasse lorsque la neutralisation de la phase glycérineuse A, dans l'étape (d), se fait au moyen d'acide phosphorique. Dans ce cas; la phase glycérineuse contiendra du phosphate de potassium, que l'on pourra séparer de la glycérine et utiliser par exemple comme engrais. Cette séparation pourrait se faire après l'étape de séchage, par exemple par filtration du sel.

La température mise en jeu pour réaliser la réaction de transestérification, en présence d'un catalyseur alcalin, peut aller par exemple de 5 à 120°C. Toutefois, il est commode de travailler entre 20 et 50°C. Si l'on souhaite économiser un réacteur, on peut réaliser la réaction dans une simple cuve de stockage, à condition de pouvoir agiter au moins quelques minutes jusqu'a l'obtention d'une phase homogène. A des températures de 5 à 8°C, on peut, pour certaines concentrations d'alcool éthylique, obtenir deux phases et aboutir, avec moins de 1% en poids de catalyseur, à une conversion totale de l'huile en ester (moins ce qui est transformé en savons). Cependant, cela n'implique pas nécessairement un bilan en poids très intéressant, car une partie de l'ester éthylique reste dans la phase glycérineuse. Par contre, à partir de 15-20°C, on obtient généralement une seule phase, surtout lorsqu'on utilise plus de 40% en poids d'alcool éthylique par rapport à l'huile de départ. Les températures plus élevées favorisent plutôt la saponification que la transestérification. Une perte d'esters éthyliques provient du fait que lorsqu'il se forme une phase glycérineuse - quelles que soient les conditions, qui sont cependant toujours basiques-, les savons entraînent dans celle-ci une quantité importante d'esters. Mais il s'agit d'une perte apparente, puisque ces esters seront récupérés ultérieurement et recyclés dans le procédé.

Le temps de réaction dépend de la température. Il peut fluctuer par exemple entre 30 minutes et 24 heures. La plupart du temps, on obtient 80% de conversion au bout de quelques minutes et parfois 97% après quelques heures. En général, il n'est pas utile d'ajouter le catalyseur en plusieurs fois, en l'absence de décantation intermédiaire.

Dans l'étape (b) du procédé de l'invention, la phase glycérineuse que l'on ajoute est avantageusement une fraction de la phase glycérineuse B produite dans la phase (d), après séchage, sauf bien entendu pour le traitement du premier lot, pour lequel on utilise une glycérine du commerce, par exemple à 90% de pureté.

La quantité de glycérine ajoutée l'étape (b) dépend de la quantité d'alcool éthylique engagée dans l'étape (a). Cependant, on ajoute en général de 20 à 40% en poids de glycérine par rapport à l'huile de départ, notamment lorsqu'il s'agit d'une huile à prédominance oléique, ou d'une autre huile contenant de manière prépondérante des acides gras à 18 atomes de carbone.

Avant l'addition de la phase glycérineuse, dans l'étape (b), on a intérêt à vérifier dans certains cas l'absence de base forte dans le milieu réactionnel. En effet, la présence d'une base forte provoquerait la réaction inverse de transestérification de l'ester éthylique par la glycérine pour re-former des glycérides. La détermination de cette basicité peut se faire aisément au pH-mètre.

L'évaporation de l'alcool éthylique, après l'ajout de glycérine dans l'étape (b), peut se faire à pression atmosphérique au moyen d'une colonne à distiller, ayant un petit nombre de plateaux (par exemple de 1 à 5), ou sous pression réduite. Dans la distillation, les premières gouttes sont de l'alcool éthylique anhydre. On arrête en général la distillation lorsqu'on a recueilli de 70 à 90% de l'excès d'alcool éthylique engagé. Il convient de s'assurer, par une méthode appropriée, que le degré alcoolique de l'alcool ainsi récupéré n'est pas inférieur à celui de l'alcool engagé au départ. Dans ces conditions, l'alcool récupéré peut être recyclé à l'entrée de l'étape (a) de transestérification.

Dans l'étape (c) du procédé de l'invention, la décantation des deux phases obtenues à l'issue de l'étape (b) est effectuée de préférence à température élevée, par exemple environ 75°C. A une telle température, trente minutes suffisent pour clarifier les deux phases. La phase ester, qui est la phase supérieure, est séparée et soumise à une purification par des moyens classiques, tels que ceux décrits par exemple dans le brevet FR-B-2.635.520 au nom du même déposant. On peut opérer par addition de 1 à 2% en poids d'eau, puis d'eau acidulée et de nouveau d'eau ; ou par passage sur résine échangeuse d'ions ou sur terre activée et évaporation ; ou encore par un couplage de différentes techniques.

Dans l'étape (d), on traite la phase glycérineuse A de manière à préparer le recyclage des corps gras (esters et acides) entraînés dans celle-ci. Les savons présents dans ladite phase glycérineuse sont neutralisés par une solution d'acide minéral fort, par exemple d'acide sulfurique, chlorhydrique ou phosphorique. Ce dernier acide permet d'éviter les problèmes de corrosion du matériel. Il se forme deux phases : la phase supérieure consiste en un mélange d'acides gras et d'esters et la phase inférieure est la phase glycérineuse B, qui contient en outre des traces d'alcool, de l'eau et les sels minéraux formés avec l'acide utilisé. Les deux phases décantent aisément, en général à une température de 30 à 50°C. Le pH de la solution glycérineuse B est en général de 5 à 6. Une petite fraction de la phase glycérineuse A peut être mise de côté pour servir de catalyseur dans l'étape suivante (e) de glycérolyse des esters et acides gras séparés. Selon la dilution de l'acide sulfurique ou phosphorique utilisé, on peut aussi obtenir directement après neutralisation une phase solide de sels, que l'on peut filtrer avant décantation.

Dans l'étape (e), on transforme les acides gras et éventuellement une partie des esters en glycérides, par glycérolyse au moyen de glycérine qui peut être une fraction de la phase glycérineuse B séparée lors de l'étape précédente (d) et séchée par exemple à une température pouvant aller jusqu'à environ 170°C. La glycérolyse est réalisée en présence d'un catalyseur alcalin, qui peut avantageusement consister en une fraction de la phase glycérineuse A (qui est alcaline puisqu'elle contient des savons), utilisée à une concentration équivalant en général à 0,1 à 0,2% en poids de soude, par rapport à la phase "acides gras + esters". Dans cette étape de glycérolyse, il n'est en général pas utile de mettre en jeu de grandes quantités de glycérine neutre et de glycérine alcaline, le but de l'opération étant seulement de réduire l'acidité du mélange "acides gras + esters" et de le transformer en glycérides. La réaction de glycérolyse est effectuée en général dans un réacteur muni d'un évaporateur et d'un condenseur pour récupérer l'eau et l'alcool présents. Elle démarre à environ 170°C et peut être menée avantageusement à une température de 180 à 220°C jusqu'à obtenir un indice d'acide inférieur à 2. La durée de la réaction dépend de la quantité d'acides gras initialement présente. On peut réaliser la réaction sous un léger vide. Le produit final est généralement formé de deux phases : une phase liquide qui est un mélange de glycérides et d'esters et une phase solide constituée de sels et de traces de savons. Cette dernière est filtrée avec adjonction d'une petite quantité d'alcool éthylique, si nécessaire.

La réaction de glycérolyse peut être réalisée en continu avec trois réacteurs en série. Cette réaction a déjà été décrite par exemple par le même déposant dans le brevet français FR-B-2.696.185.

Si leur acidité est réduite à un indice d'acide inférieur à 2, rien ne s'oppose - sauf parfois leur couleur - à ce que les glycérides ainsi formés soient utilisés tels quels comme seule matière première dans une nouvelle fabrication d'esters. Cependant, on a intérêt à les mélanger à l'huile de départ par retour vers l'étape (a) du procédé de l'invention. Compte tenu du fait que la phase "acide + esters" récupérée et traitée par glycérolyse représente en général de 15 à 20 % en poids de la charge initiale à transestérifier, si l'on suppose que, dans la pratique, on utilise pour la glycérolyse et pour la transestérification un réacteur de même contenance, on aura à effectuer un traitement de glycérolyse chaque fois que l'on aura réalisé d'environ 5 à 7 opérations de transestérification. En ce qui concerne le bilan du procédé de l'invention, on peut obtenir entre 100 et 105% en poids d'esters éthyliques par rapport à l'huile de départ. Les pertes sont donc négligeables.

Au cours du procédé, de l'eau est éliminée et/ou séparée à trois stades. Tout d'abord dans l'étape (b), lorsqu'on évapore l'alcool en obtenant un azéotrope ou même de l'alcool enrichi ; puis dans l'étape (d), lorsque l'on sèche la phase glycérineuse B ; enfin, dans l'étape (e), lors de la glycérolyse.

Si l'on fait le bilan de la glycérine dans le procédé, on peut dénombrer quatre flux de glycérine. Trois flux sont issus de la phase glycérineuse B séchée : celui qui est recyclé pour améliorer la décantation du millieu réactionnel après la réaction de transestérification dans l'étape (b) ; celui qu'on envoie vers la glycérolyse de l'étape (e); et une fraction majoritaire que l'on sort du procédé. Un quatrième flux de glycérine est celui issu de la phase glycérineuse A qui peut être utilisé pour apporter le catalyseur basique dans la glycérolyse.

Si l'on considére le bilan de l'alcoool éthylique, on constate que la proportion d'alcool perdue au cours du procédé est faible, car la glycérine, dans l'étape (b), permet de récupérer un alcool peu hydraté, la présence de glycérine ayant pour effet d'inhiber l'évaporation de l'eau, donc de favoriser celle de l'alcool.

Par ailleurs, le mélange "eau + alcool" récupéré lors du séchage de la glycérine B dans l'étape (d) et/ou après glycérolyse dans l'étape (e) peut servir par exemple dans le lavage de résines échangeuses d'ions qu'il est possible d'utiliser pour purifier l'ester obtenu à l'issue de la phase (c).

Les exemples suivants illustrent l'invention.

### Exemple 1. Fabrication d'esters éthyliques d'huile de tournesol.

Dans une première étape, on mélange 1000 g d'huile de tournesol neutralisée, 500 g d'alcool éthylique à 95% en poids d'alcool et 15 g de soude préalablement dissous dans de l'alcool hydraté. La réaction de transestérification est réalisée sous agitation à 30°C. Après quelques minutes, la solution trouble devient homogène. Après 2 heures, on procède au prélèvement d'un échantillon de la solution et l'on constate par chromatographie en phase liquide (GPC : abréviation de l'anglais "Gas Permeation Chromatography") que l'ester a une pureté de 98%, le reste étant constitué de mono-, di- et triglycérols et d'esters de stérols.

On vérifie l'absence d'alcalis forts dans le milieu avant de procéder à une deuxième étape, dans laquelle on ajoute 300 g de glycérine contenant 5% en poids d'alcool et 5% en poids d'eau. On évapore le mélange de glycérine, d'ester et d'alcool à la pression atmosphérique dans une colonne à distiller et l'on recueille 300g d'alcool titrant 95,3%. Au début de la distillation, l'alcool qui sort est sec, puis il est de plus en plus hydraté. La température en haut de la colonne de distillation est de 78°C et en bas, elle est de 120°C. On refroidit à 75°C les deux phases formées et l'on décante. On obtient 620 g de phase glycérineuse (phase inférieure) et 895 g de phase ester (phase supérieure), qui contient encore de l'alcool dissous.

Dans une troisième étape, on lave la phase ester et on la sèche. On obtient 857 g d'un ester sec qui présente une pureté proche de celle de l'échantillon prélevé précédemment. La pureté est de 97,6%.

Dans une quatrième étape, on neutralise la phase glycérineuse par 40 cm³ d'acide chlorhydrique et l'on obtient deux phases, l'une glycérineuse (451 g), l'autre constituée d'un mélange d'acides gras et d'esters (209 g). On assure ainsi la séparation entre la fraction "acides gras + esters" et la glycérine.

Dans une cinquième étape, on effectue la transestérification ou glycérolyse des acides gras et/ou des esters avec la glycérine en chauffant la fraction "acides gras + esters" avec de la glycérine neutralisée et une fraction basique non neutralisée de la glycérine, en utilisant en fait comme catalyseurs les savons présents dans la glycérine et qui sont des catalyseurs aux températures supérieures à, par exemple, 170°C.

Pour optimiser les rendements, on recueille les acides gras + esters obtenus dans deux transestérifications successives. Alors que dans la première transestérification, on utilisait pour provoquer la décantation une glycérine du commerce, pour le second lot d'ester, on utilise la glycérine neutralisée obtenue dans la première réaction. En pratique, on chauffe 400 g de la phase ester + acides gras obtenue dans les deux réactions avec un mélange de 31 g de glycérine basique non neutralisée qui contient 0,75 g de soude potentielle sous forme de savons et 100 g de glycérine neutralisée à l'acide chlorhydrique jusqu'à un pH de 6.

Les 100 g de glycérine proviennent d'un lot de 430 g de glycérine neutralisée chauffée à 150°C pour la sécher partiellement et dont on a prélevé 300 g pour la troisième étape et la troisième réaction.

On chauffe à 180-200°C sous pression atmosphérique. Il se dégage quelques dizaines de grammes d'un mélange alcool-eau. On titre après 3 heures de chauffage le mélange, qui présente un indice d'acide inférieur à 2. On filtre les sels.

455 g de glycérides et 545 g d'huile sont soumis à une transestérification comme dans l'exemple 1. On obtient à nouveau 860 g d'ester. Le rendement final est donc, en poids par rapport à l'huile, de (857 + 860 + 860) / (1000 + 1000 + 545) = 101,5%.

Les pertes sont principalement des savons et de l'ester qui, lors de la filtration du sel de la phase "esters + glycérides", restent imprégnés sur celui-ci. On perd aussi de l'ester dans le lavage de l'ester et sa purification. Le rendement est toutefois très satisfaisant.

Les esters éthyliques obtenus présentent la composition globale suivante :
- esters éthyliques : 95,87%
- monoglycérides : 1,90 %
- diglycérides + esters de stérols : 2,06 %
- triglycérides : 0,17 %
- alcalinité : < 5 ppm

### Exemple 2.

Dans cet exemple, pour mettre en évidence l'influence de la quantité d'alcool éthylique engagé sur la pureté des esters obtenus, on répète plusieurs fois la procédure de l'exemple 1. Dans les divers essais, on introduit successivement dans 100 g d'huile de tournesol des quantités d'alcool éthylique à 95% en poids allant de 30 à 100 g. La quantité de soude dissoute dans l'alcool est de 1,5 g.

A 30°C, on obtient au bout d'une heure une phase homogène. La pureté des esters après élimination de l'alcool et du catalyseur est donnée par le tableau suivant :

| Quantité d'alcool (g) | MG* (% poids) | DG* (% poids) | TG* (% poids) | EE* (% poids) |
|---|---|---|---|---|
| 30 | 7,5 | 4,5 | 3,4 | 84,5 |
| 40 | 6,3 | 4,5 | 3,4 | 85,7 |
| 50 | 1,3 | 3,6 | 0,8 | 94,0 |
| 100 | 0,9 | 1,8 | 0,0 | 97,2 |

| | | | | |
|---|---|---|---|---|
| * MG = monoglycérides, DG = diglycérides + esters de stérols, TG = triglycérides, EE = esters éthyliques. | | | | |

On obtient de bons résultats pour des poids d'alcool de 50 et de 100 g.

### Exemple 3.

Dans cet exemple, on répète plusieurs fois la procédure de l'exemple 1, en opérant avec un rapport pondéral alcool / huile de 1, une concentration en soude de 1,5% en poids par rapport à l'huile, avec agitation au départ de quelques minutes jusqu'à l'obtention d'une phase homogène, puis poursuite de la réaction 24 heures à 20°C. On fait varier la teneur en eau de l'alcool éthylique utilisé. Les résultats sont donnés dans le tableau suivant :

| eau dans l'alcool (% poids) | agitation (min) | MG (%poids) | DG (%poids) | TG (%poids) | EE (%poids) |
|---|---|---|---|---|---|
| 3 | 0,6 | 1,20 | 1,8 | 0 | 97,0 |
| 5 | 3 | 1,0 | 1,8 | 0 | 97,2 |
| 8 | 5 | 1,3 | 2,1 | 0,4 | 96,2 |
| 10 | 8 | 3,4 | 4,4 | 13,1 | 79,0 |

On met ainsi en évidence que, lorsque la quantité d'alcool introduite est importante par rapport à l'huile, on peut réaliser la réaction de transestérification avec des teneurs en eau dans l'alcool qui peuvent être supérieures à 5% en poids.

### Exemple 4.

On réalise des essais analogues à ceux de l'exemple 3, mais avec 50% en poids d'alcool éthylique par rapport à l'huile. On opère à 30°C pendant 1 heure. Les résultats sont donnés dans le tableau suivant, qui montre que l'on obtient de moins bonnes performances.

| eau dans l'alcool (% poids) | MG (% poids) | DG (% poids) | TG (% poids) | EE (% poids) |
|---|---|---|---|---|
| 5 | 1,3 | 3,7 | 0,8 | 94 |
| 7 | 5,5 | 3,9 | 3,4 | 85 |
| 10 | 9,6 | 12,8 | 21 | 56,2 |

### Exemple 5

On répète plusieurs fois la procédure de l'exemple 1 en faisant varier la teneur en eau de la glycérine ajoutée après la réaction de transestérification. L'alcool éthylique utilisé présente un taux d'alcool de 95% en poids. On détermine la teneur en alcool éthylique de l'alcool qui passe en début de distillation et le rendement final en esters éthyliques. Les résultats sont indiqués dans le tableau suivant :

| eau dans la glycérine introduite (% poids) | teneur en alcool de l'alcool distillé (%poids) | rendement en esters (%poids) |
|---|---|---|
| 0 | 99,9 | 85,0 |
| 20 | 96,6 | 85,0 |
| 35 | 91,6 | 85,1 |
| 50 | 89,4 | 84,4 |

Il apparait que le rendement poids en esters ne dépend pas de manière significative de la quantité d'eau présente dans la glycérine ajoutée. Par contre, celle-ci a une influence sur la teneur en eau de l'alcool récupéré en début de distillation, même si l'on utilise un alcool éthylique à 95% en poids.

## Revendications

1. Procédé de fabrication d'esters éthyliques d'acides gras à partir d'huile ou de graisse végétale ou animale ou autres mélanges de glycérides, caractérisé en ce qu'il comprend :
- une étape (a) dans laquelle on transestérifie l'huile, la graisse ou le mélange de glycérides de départ par de l'alcool éthylique hydraté en excès en utilisant un catalyseur alcalin ;
- une étape (b) dans laquelle on ajoute dans le milieu une phase glycérineuse et on évapore l'alcool éthylique en excès, de manière à produire deux phases, une phase ester et une phase glycérineuse A, non miscibles, et l'on recycle l'alcool éthylique en excès vers l'étape (a) ;
- une étape (c) dans laquelle on sépare ladite phase glycérineuse A et ladite phase ester, que l'on purifie, de manière à obtenir les esters éthyliques recherchés ;
- une étape (d) dans laquelle on neutralise ladite phase glycérineuse A et on sépare une phase "acides gras + esters" et une phase glycérineuse B, que l'on sèche ;
- et une étape (e) dans laquelle on effectue la glycérolyse de la phase "acides gras + esters" avec au moins une fraction de la phase glycérineuse B séchée et en présence d'un catalyseur alcalin, pour former un mélange de glycérides et d'esters, que l'on envoie dans une étape de transestérification telle que (a).

2. Procédé selon la revendication 1 dans lequel la réaction de transestérification de l'étape (a) est réalisée à une température de 5 à 120°C avec, comptées par rapport à l'huile de départ, une proportion de catalyseur alcalin correspondant à 1 à 2% en poids de soude et une proportion de 30 à 100% en poids d'alcool éthylique hydraté présentant une teneur en alcool de 92 à 99% en poids.

3. Procédé selon l'une des revendications 1 et 2 dans lequel l'alcool éthylique hydraté utilisé présente une teneur en alcool de 94 à 97% en poids et est utilisé en une proportion de 40 60% en poids par rapport à l'huile de départ.

4. Procédé selon l'une des revendications 1 à 3 dans lequel l'excès d'alcool éthylique de l'étape (b) est évaporé jusqu'à obtenir un taux d'alcool éthylique au minimum égal à celui de l'alcool de départ pour être recyclé vers l'étape (a).

5. Procédé selon l'une des revendications 1 à 4 dans lequel la phase glycérineuse utilisée dans l'étape (b) est une fraction de la phase glycérineuse B obtenue à l'étape (d) séchée jusqu'à une température d'environ 150°C.

6. Procédé selon la revendication 5 dans lequel ladite phase glycérineuse contient de 1 à 20% d'eau et est utilisée en une proportion de 20 à 40% par rapport à l'huile de départ.

7. Procédé selon l'une des revendications 1 à 6 dans lequel la réaction de glycérolyse de l'étape (e) est effectuée à une température de 170 à 220°C, catalysée au moyen d'une fraction de la phase glycérineuse basique A séparée dans l'étape (c) et poursuivie jusqu'à obtention d'un produit dont l'indice d'acide est inférieur à 2.

8. Procédé selon l'une des revendications 1 à 7 dans lequel le produit obtenu à l'issue de l'étape (e) est, après filtration, recyclé vers l'huile, la graisse ou le mélange de glycérides de départ ou vers l'étape (a).

## Patentansprüche

1. Verfahren zur Herstellung von Fettsäureäthylestern auf der Grundlage von pflanzlichem oder tierischem Öl oder Fett oder von anderen Glyzeridgemischen, dadurch gekennzeichnet, dass es sich wie folgt zusammensetzt:
- einer Stufe (a), in der das Ausgangsöl, -fett oder - glyzeridgemisch durch einen überschüssigen, wasserhaltigen Äthylalkohol unter Verwendung eines alkalischen Katalysators transverestert wird;
- einer Stufe (b), in der eine glyzerinhaltige Phase in das Milieu addiert wird, man den überschüssigen Äthylalkohol verdunstet, um zwei Phasen zu erhalten, eine Esterphase und eine glyzerinhaltige Phase A, die miteinander nicht mischbar sind, und man den überschüssigen Äthylalkohol zur Stufe (a) zurückführt;
- einer Stufe (c), in der die besagte glyzerinhaltige Phase A und die besagte Esterphase, die zur Gewinnung der gewünschten Äthylester gereinigt wird, getrennt werden.
- einer Stufe (d), in der man die besagte glyzerinhaltige Phase A neutralisiert und eine "Fettsäuren+Ester"-Phase sowie eine glyzerinhaltige Phase B abtrennt, die getrocknet wird;
und einer Stufe (e), in der die Glyzerolyse der "Fettsäuren+Ester"-Phase mit mindestens einem Teil der getrockneten glyzerinhaltigen Phase B in Gegenwart von einem alkalischen Katalysator erfolgt, um ein Gemisch aus Glyzeriden und Estern zu erhalten, das einer Transveresterungsstufe gemäß (a) zugeführt wird.

2. Verfahren nach Anspruch 1, in dem die Transveresterungsreaktion aus Stufe (a) bei einer Temperatur von 5 bis 120°C erfolgt, wobei im Verhältnis zum Ausgangsöl der Anteil des alkalischen Katalysators von 1 bis 2 Gewichtsprozent Natriumcarbonat und der Anteil des wasserhaltigen Äthylakohols, der einen Alkoholgehalt von 92 bis 99 Gewichtsprozent aufweist, von 30 bis 100 Gewichtsprozent beträgt.

3. Verfahren nach einem der Ansprüche 1 und 2, in dem der verwendete wasserhaltige Äthylalkohol einen Alkoholgehalt von 94 bis 97 Gewichtsprozent aufweist und mit einem Anteil von 40 bis 60 Gewichtsprozent im Verhältnis zu Ausgangsöl eingesetzt wird.

4. Verfahren nach einem der Ansprüche 1 bis 3, in dem der überschüssig eingesetzte Äthylalkohol aus Stufe (b) verdunstet wird, bis man einen Äthylalkoholgehalt erzielt, der mindestens gleich mit dem Alkoholgehalt des Ausgangsalkohols ist, um zur Stufe (a) zurückgeführt zu werden.

5. Verfahren nach einem der Ansprüche 1 bis 4, in dem die Glyzerinphase, die in Stufe (b) Verwendung findet, eine Fraktion der glyzerinhaltigen Phase B ist, die in Stufe (d) gewonnen und bei einer Temperatur von bis etwa 150°C getrocknet wurde.

6. Verfahren nach dem Anspruch 5, in dem die besagte glyzerinhaltige Phase 1 bis 2 Prozent Wasser enthält und in einem Verhältnis von 20 bis 40 Prozent zum Ausgangsöl eingesetzt wird.

7. Verfahren nach einem der Ansprüche 1 bis 6, in dem die Glyzerolysereaktion aus Stufe (e) bei einer Temperatur von 170 bis 220°C erfolgt, wobei als Katalysator eine Fraktion der basischen, glyzerinhaltigen Phase A, die in Stufe (c) getrennt wurde, dient, und bis zur Gewinnung eines Produktes mit einem Säurewert von weniger als 2 fortgesetzt wird.

8. Verfahren nach einem der Ansprüche 1 bis 7, in dem das am Ende von Stufe (e) erzeugte Produkt nach Filtration zum Ausgangsöl, Ausgangsfett oder Ausgangsglyzeridgemisch oder auch zur Stufe (a) zurückgeführt wird.

## Claims

1. Process for the production of ethyl esters of fatty acids from vegetable or animal oil or fat or other mixtures of glycerides, characterized in that it comprises:
- a step (a) of transesterifying fatty acid glycerides with hydrated ethyl alcohol using an alkaline catalyst to form a medium comprising ethyl esters and excess ethyl alcohol;
- a step (b) of adding a glycerine phase to said medium, and evaporating the excess ethyl alcohol to produce two immiscible phases, an ester phase and a glycerine phase A, and recycling said excess ethyl alcohol to step (a);
- a step (c) of separating said glycerine phase A and said ester phase to obtain the desired ethyl esters;
- a step (d) of neutralizing said glycerine phase A with acid, and separating resultant "fatty acids + esters" phase and a glycerine phase B, and drying the latter phase;
- and a step (e) of subjecting the "fatty acids + esters" phase to glycerolysis with at least a fraction of the dried glycerine phase B in the presence of an alkaline catalyst to form a mixture of glycerides and esters, and passing said mixture into transesterification step (a).

2. Process according to claim 1, wherein the transesterification reaction of step (a) is carried out at a temperature of 5 to 120°C with, computed relative to the starting oil, a proportion of alkaline catalyst corresponding to 1 to 2% by weight of soda, and a proportion of 30 to 100% by weight of hydrated ethyl alcohol that exhibits an alcohol content of 92 to 99% by weight.

3. Process according to one of claims 1 and 2, wherein the hydrated ethyl alcohol that is used exhibits an alcohol content of 94 to 97% by weight and is used at a proportion of 40 to 60% by weight relative to the starting oil.

4. Process according to one of claims 1 to 3, wherein the excess ethyl alcohol of step (b) is evaporated to obtain an ethyl alcohol level that is at least equal to that of the starting alcohol to be recycled to step (a).

5. Process according to one of claims 1 to 4, wherein the glycerine phase that is used in step (b) is a fraction of glycerine phase B that is obtained in step (d), dried to a temperature of about 150°C.

6. Process according to claim 5, wherein said glycerine phase contains 1 to 20% water and is used at a proportion of 20 to 40% relative to the starting oil.

7. Process according to one of claims 1 to 6, wherein the glycerolysis reaction of step (e) is carried out at a temperature of 170 to 220°C, catalyzed with a fraction of basic glycerine phase A that is separated in step (c) and is continued until a product is obtained whose acid number is less than 2.

8. Process according to one of claims 1 to 7, wherein the product that is obtained at the end of step (e) is, after filtration, recycled to the oil, the fat, or the starting mixture of glycerides or to step (a).
